# EUROPÄISCHE PATENTSCHRIFT

(11) **EP 1 035 093 B1**
(45) Veröffentlichungstag und Bekanntmachung des Hinweises auf die Patenterteilung: **10.09.2003**
(21) Anmeldenummer: 00103339.8
(22) Anmeldetag: 21.02.2000
(51) Int. Cl.: C07B 37/10, C08F 2/14, C07D 407/04, C07C 13/20, C07C 271/24, C07D 225/02, C07D 295/185, C07F 7/08

(54) **Metathese in Gegenwart ionischer Flüssigkeiten**
Metathesis in the presence of ionic liquids
Métathèse en présence de liquides ioniques

(30) Priorität: 05.03.1999 DE 19909600; 18.06.1999 DE 19927912
(43) Veröffentlichungstag der Anmeldung: 13.09.2000
(73) Patentinhaber: BAYER AG, 51368 Leverkusen (DE)
(72) Erfinder: Gürtler, Chistoph, Dr., 50676 Köln (DE); Jautelat, Manfred, Dr., 51399 Burscheid (DE)

(56) Entgegenhaltungen:
- EP-A- 0 882 691
- US-A- 5 110 885
- S. K. ARMSTRONG: "Ring closing diene metathesis in organic synthesis" JOURNAL OF THE CHEMICAL SOCIETY, PERKIN TRANSACTIONS 1, 1998, Seiten 371-388, XP002153812 LETCHWORTH GB

## Beschreibung

Die vorliegende Erfindung betrifft ein Verfahren zur Herstellung cyclischer und/oder polymerer Verbindungen durch Metathese von Edukten, die mindestens zwei funktionelle Gruppen in Form von substituierten oder unsubstituierten Alken- oder Alkineinheiten enthalten.

Unter Metathese versteht man eine wechselseitige Umalkylidenierung von Alkenen und Alkinen in Gegenwart von Katalysatoren. Reaktionen dieser Art finden Anwendungen in einer Vielzahl technisch wichtiger Prozesse. Eine Übersicht hierüber findet sich in: M. Schuster, S. Blechert, *Angew. Chem*. **1997**, *109*, 2124 und S. Armstrong, *J. Chem. Soc., Perkin Trans. 1*, **1998**, 371. Zu den Metathesereaktionen zählen die Oligomerisation und Polymerisation von acyclischen Dienen beispielsweise in US 5 110 885 (ADMET) und die Synthese von Carbo- und Heterozyklen unterschiedlicher Ringgrößen durch Ringschlußmetathese (RCM, Ring-Closing-Metathesis). Darüberhinaus sind gekreuzte Metathesen unterschiedlicher Alkene bekannt (Brümmer, O. et al. *Chem. Eur. J.* **1997**, *3*, 441).

Für die oben erwähnten Metathesereaktionen können die in WO-A-93/20111 beschriebenen Ruthenium-Alkylidenverbindungen, die von A. W. Stumpf, E. Saive, A. Deomceau und A. F. Noels in *J. Chem. Soc., Chem. Commun*. **1995**, 1127-1128 beschriebenen Katalysatorsysteme auf Rutheniumbasis oder die von P. Schwab, R. H. Grubbs und J. W. Ziller in *J. Am. Chem. Soc*. **1996**, *118*, 100 (siehe auch WO 96/04289) beschriebenen Katalysatorsysteme als Katalysatoren eingesetzt werden.

In jüngerer Zeit wurde die Verwendung von sogenannten nicht wässrigen ionischen Flüssigkeiten für Metathesereaktionen beschrieben.

Bei ionischen Flüssigkeiten handelt es sich um Salze oder Mischungen von Salzen, die in einem weiten Temperaturbereich flüssig sind. Der Vorteil ionischer Flüssigkeiten liegt darin, dass sie sich nicht mit aliphatischen Kohlenwasserstoffen mischen.

Bei organischen Reaktionen, für die der Einsatz eines Katalysators notwendig ist, kann man durch Zusatz von ionischen Flüssigkeiten und eines geeigneten Katalysators, der sich nur oder bevorzugt in der ionischen Flüssigkeit löst, eine heterogene Katalyse erreichen.

US-A-5,104,840 beschreibt den Einsatz solcher Mischungen als Lösungsmittel für Übergangsmetallkomplexe, speziell Nickel-Komplexe, die keine Kohlenstoff-Nickel-Bindungen enthalten.

In EP-B-448445 wird der Einsatz von ionischen Flüssigkeiten für die Dimerisierung von unsubstituierten Monoolefinen unter Verwendung von Nickelchlorid beschrieben. Nachteilig ist hierbei allerdings die Verwendung von Organoaluminiumhalogenid, speziell des pyrophoren Dichloroethylaluminiums, zur Herstellung der ionischen Flüssigkeit.

US-A-5525567 beschreibt den Einsatz von ionischen Flüssigkeiten für die Disproportionierung von unsubstituierten Monoolefinen unter Verwendung von Wolframkatalysatoren. Auch hier wird obligatorisch ein Organoaluminiumhalogenid, bevorzugt das pyrophore Dichloroethylaluminium, zur Herstellung der ionischen Flüssigkeit eingesetzt.

EP-A-882691 beschreibt den Einsatz von ionischen Flüssigkeiten für die Dimerisierung von unsubstituierten Monoolefinen unter Verwendung von Nickelchlorid. Neben dem auch hier verwendeten pyrophoren Dichloroethylaluminium ist weiterhin nachteilig, daß durch den Überschuß an der Lewis-Säure Aluminiumchlorid in der ionischen Flüssigkeit eine saure Reaktionsmischung entsteht.

Die oben beschriebenen Metatheseverfahren eignen sich allerdings nur für die Umsetzung von unsubstitiuierten Monoolefinen, also sehr einfachen organischen Molekülen. Für die Umsetzung von mehrfach substituierten Edukten mit funktionellen Gruppen sind diese Verfahren nicht geeignet, da die dort beschriebenen Katalysatoren nicht anwendbar sind oder die ionischen Flüssigkeiten aufgrund ihrer Zusammensetzung oder der Tatsache, daß die Reaktionsmischung sauren Charakter aufweist, für andere Katalysatoren nicht geeignet sind.

Aufgabe der vorliegenden Erfindung war es, ein universell einsetzbares Verfahren zur Metathese von Edukten, die mindestens zwei funktionelle Gruppen in Form von Alken- oder Alkineinheiten enthalten, bereitzustellen, das unter Verwendung von ionischen Flüssigkeiten durchgeführt wird. Das Verfahren sollte auch für substituierte Alkene oder Alkine einsetzbar sein.

Es wurde ein Verfahren zur Herstellung cyclischer und/oder polymerer Verbindungen durch Metathese von Edukten, die mindestens zwei funktionelle Gruppen in Form von substituierten oder unsubstituierten Alken- oder Alkineinheiten enthalten, in Gegenwart von einem oder mehreren homogen oder heterogen vorliegenden Katalysatoren gefunden, das dadurch gekennzeichnet ist, daß die Metathese in Gegenwart von ionischen Flüssigkeiten durchgeführt wird und daß als Katalysatoren Übergangsmetallcarbene oder Übergangsmetallverbindungen, die unter den Reaktionsbedingungen Übergangsmetallcarbene bilden oder Übergangsmetallsalze in Verbindung mit einem Alkylierungsmittel verwendet werden.

Überraschenderweise wurde gefunden, daß die Gegenwart von ionischen Flüssigkeiten bei Metathesereaktion von Edukten, die mindestens zwei funktionelle Gruppen in Form von substituierten oder unsubstituierten Alken- oder Alkineinheiten enthalten dazu führt, daß die Standzeit des Katalysators verlängert wird, da er in der ionischen Flüssigkeit für weitere Metathesereaktionen eingesetzt werden kann.

In einer bevorzugten Ausführungsform betrifft die vorliegende Erfindung die Herstellung carbo- oder heterocyclischer Verbindungen mit Ringgrößen ≥ 5 Ringglieder, einschließlich der mittleren (8 bis 11 Ringglieder) und großen (≥ 12 Ringglieder) Ringe und/oder die Herstellung polymerer Verbindungen, bei denen es sich um Homopolymere, Copolymere oder Block-Copolymere handeln kann.

Bei Ringschlußmetathesereaktionen steht die Ringschlußreaktion in Konkurrenz zur Polymerisation. Führt man diese Reaktion mit Edukten durch, die mindestens zwei funktionellen Gruppen in Form von Alken- oder Alkineinheiten enthalten, so entstehen Gemische aus cyclischen Verbindungen und Polymeren.

Die Bildung von cyclischen Verbindungen wird durch die Durchführung der Reaktion in organischen Lösungsmitteln bei hoher Verdünnung oder durch die Zugabe größerer Volumina an ionischen Flüssigkeiten begünstigt. Dies gilt insbesondere für die Herstellung von mittleren (8 bis 11 Ringglieder) und großen (≥ 12 Ringglieder) Ringen.

Die zur Erreichung der notwendigen hohen Verdünnung erforderlichen großen Reaktionsvolumina an organischen Lösungsmitteln limitieren die maximalen Raum/Zeit-Ausbeuten. Die Abtrennung der Produkte nach Beendigung der Reaktion erfordert zeitaufwendige Trennoperationen wie Chromatographie und führt meist zu einer irreversiblen Desaktivierung des verwendeten Katalysators.

Durch Verwendung von größeren Volumina an ionischen Flüssigkeiten lassen sich die gewünschten Produkte allerdings leicht abtrennen, da sie sich in der organischen Phase befinden, die mit der ionischen Flüssigkeit nicht mischbar ist. Wählt man einen Katalysator, der sich ausschließlich oder bevorzugt in der ionischen Flüssigkeit löst, so läßt sich die Desaktivierung des Katalysators nach der Aufarbeitung vermeiden und die Phase, die die ionische Flüssigkeit und den Katalysator enthält, kann für weitere Metathesereaktionen eingesetzt werden.

Bevorzugt werden im erfindungsgemäßen Verfahren Edukte eingesetzt, die neben den an der Metathesereaktion beteiligten funktionellen Gruppen mindestens einen weiteren, sich in der Metathesereaktion inert verhaltenden Substituenten und/oder ein Heteroatom enthalten. Diese Substituenten oder Heteroatome können dabei unabhängig gewählt werden aus: verzweigte oder unverzweigte Alkylreste, aromatische oder nicht-aromatische carbocyclische Ringe, Carbonsäuren, Ester, Ether, Epoxide, Silylether, Thioether, Thioacetale, Anhydride, Imine, Silylenolether, Ammoniumsalze, Amide, Nitrile, Perfluoralkyl-Gruppen, gem.-Dialkyl-Gruppen, Alkine, Alkene, Halogene, Alkohole, Ketone, Aldehyde, Carbamate, Carbonate, Urethane, Sulfonate, Sulfone, Sulfonamide, Nitro-Gruppen, Organosilan-Einheiten, Metallzentren, sauerstoff-, stickstoff-, schwefel-, phosphorhaltige Heterocyclen.

Besonders bevorzugt werden für das erfindungsgemäße Verfahren als Edukte α,ω-Diene eingesetzt, die mindestens einen weiteren, sich in der Metathesereaktion inert verhaltenden Substituenten und/oder ein Heteroatom enthalten können. Diese Substituenten oder Heteroatome können dabei unabhängig gewählt werden aus verzweigte oder unverzweigte Alkylreste, aromatische oder nicht-aromatische carbocyclische Ringe, Carbonsäuren, Ester, Ether, Epoxide, Silylether, Thioether, Thioacetale, Anhydride, Imine, Silylenolether, Ammoniumsalze, Amide, Nitrile, Perfluoralkyl-Gruppen, gem.-Dialkyl-Gruppen, Alkine, Alkene, Halogene, Alkohole, Ketone, Aldehyde, Carbamate, Carbonate, Urethane, Sulfonate, Sulfone, Sulfonamide, Nitro-Gruppen, Organosilan-Einheiten, Metallzentren, sauerstoff-, stickstoff-, schwefel-, phosphorhaltige Heterocyclen.

Insbesondere werden für das erfindungsgemäße Verfahren als Edukte α,ω-Diene eingesetzt, die in α-Stellung zu einer Doppelbindung einen Substituenten NRR¹ tragen, wobei
- R: ein organischer Substituent, bevorzugt Wasserstoff, gegebenenfalls anelliertes Aryl, Alkyl, CN, COOR² oder Halogen ist,
- R¹: *tert*.-Butyl, P(R)₂, P(R²)₂, COR, SO₂PhR, COOR oder CONRR² ist,
- R²: Alkyl oder Phenyl ist,
- R und R¹: gemeinsam 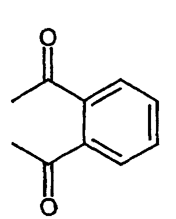 bedeuten und
besagte α,ω-Diene auch an jeder anderen Position im Molekül, mit Ausnahme der α-Stellung, mindestens einen weiteren Substituenten R tragen können.

Bei Verwendung dieser Diene als Edukte für das erfindungsgemäße Verfahren sind cyclische und/oder polymere Verbindungen erhältlich, die in α-Stellung zur Doppelbindung einen Substituenten NRR¹ tragen, wobei R und R¹ die oben angegebenen Bedeutung haben.

In einer besonders bevorzugten Ausführungsform handelt es sich bei den oben genannten α,ω-Dienen um Verbindungen der allgemeinen Formel (I) 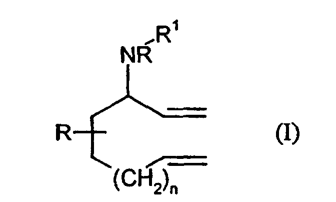 in welcher R, R¹ und R² die obengenannte Bedeutung haben und
- n: die Zahl 1,2,3 oder 4, bevorzugt 1 oder 2, besonders bevorzugt 1 bedeutet.

Bei Einsatz von Verbindungen der allgemeinen Formel (I) werden durch das erfindungsgemäße Verfahren bevorzugt cyclische Verbindungen der allgemeinen Formel (II), 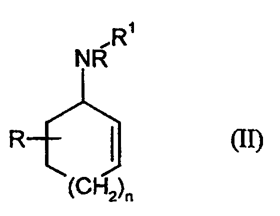 in welcher R, R¹,R² und n die obengenannte Bedeutung haben und auch die Doppelbindung durch mindestens einen Rest R substituiert sein kann, sowie polymere Produkte erhalten.

Ganz besonders bevorzugt werden für das erfindungsgemäße Verfahren als Edukte Diallylamin oder 3-Amino-1,7-octadien, besonders bevorzugt in ihrer N-Carboxymethyl-geschützten Form oder 1,7-Octadien, 10-Undecenoyl-allylamid, 1,4-Bis-oxypropen-2-yl-butin-2 oder 10-Undecensäure-buten-4-yl-ester eingesetzt.

Im erfindungsgemäßen Verfahren können auch Mischungen von Edukten eingesetzt werden. Dabei können die Edukte dem Reaktionsmedium als Gemisch zugesetzt werden oder aber dem Reaktionsmedium sequentiell zugesetzt werden.

Für das erfindungsgemäße Verfahren werden als Katalysatoren oder Katalysatorvorstufen Übergangsmetallcarbene oder Übergangsmetallverbindungen, die unter den Reaktionsbedingungen Übergangsmetallcarbene bilden oder Übergangsmetallsalze in Verbindung mit einem Alkylierungsmittel verwendet, wobei diese Katalysatoren sowohl ionisch als auch nicht-ionisch sein können.

Bevorzugt werden für das erfindungsgemäße Verfahren Katalysatoren der allgemeinen Formel (III) bis (VI) verwendet, wobei M Ruthenium oder Osmium, bevorzugt Ruthenium bedeutet. 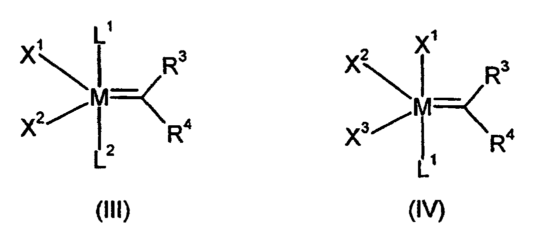 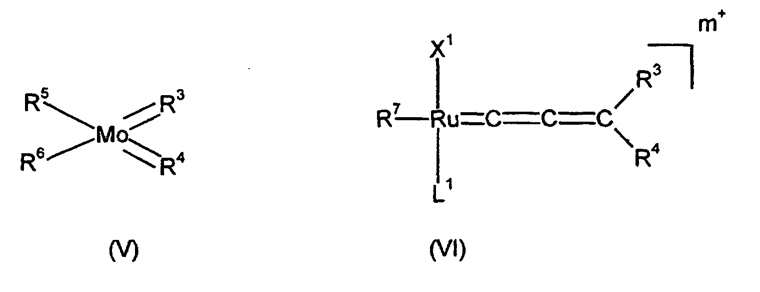
- R³ bis R⁷: sind unabhängig voneinander wählbare Reste aus Wasserstoff, C₁-C₂₀ Alkyl, C₃-C₈ Cycloalkyl, C₂-C₂₀ Alkenyl, C₂-C₂₀ Alkinyl, C₆-C₁₈ Aryl, C₁-C₂₀ Carboxylat, C₁-C₂₀ Alkoxy, C₂-C₂₀ Alkenyloxy, C₂-C₂₀ Alkinyloxy, C₆-C₁₈ Aryloxy, C₂-C₂₀ Alkoxycarbonyl, C₁-C₂₀ Alkylthio, C₁-C₂₀ Alkylsulfonyl oder C₁-C₂₀ Alkylsulfinyl, N-Aryl; jeweils wahlweise substituiert mit C₁-C₁₂ Alkyl, Perfluoralkyl, Halogen, C₁-C₅ Alkoxy oder C₆-C₁₈ Aryl. Die Reste R³ bis R⁷ können in cyclischen Verbindungen miteinander verknüpft vorliegen.
- X¹ bis X³: sind unabhängig voneinander wählbare anionische Liganden, insbesondere F⁻, Cl⁻, Br⁻, CN⁻, SCN⁻, R³O⁻, R³R⁴N⁻, (R³-R⁷)-Allyl⁻, (R³-R⁷)-Cyclopentadienyl⁻, wobei die Reste R³ bis R⁷ die bereits genannte Definition erfüllen.
- L¹ bis L³: sind unabhängig voneinander wählbare neutrale Liganden insbesondere CO, CO₂, R³NCO, R³R⁴C=CR⁵R⁶, R³C≡CR⁴, R³R⁴C=NR⁵, R³C≡N, R³OR⁴, R³SR⁴, NR³R⁴R⁵, PR³R⁴R⁵, AsR³R⁴R⁵, SbR³R⁴R⁵, wobei die Reste R³ bis R⁵ die bereits genannte Definition erfüllen.
- m: ist 1 oder 2.

Besonders bevorzugte Katalysatoren oder Katalysatorvorstufen sind Verbindungen der allgemeinen Formel (III) und (IV) mit L¹ und L² = PR³R⁴R⁵, wobei R³ bis R⁵ den oben genannten Definitionen entsprechen, ganz besonders bevorzugte Reste sind dabei Aryl oder Alkyl, insbesondere sekundäre Alkylreste oder Cycloalkylreste.

Ganz besonders bevorzugt werden als Katalysatoren für das erfindungsgemäße Verfahren folgende Verbindungen eingesetzt: 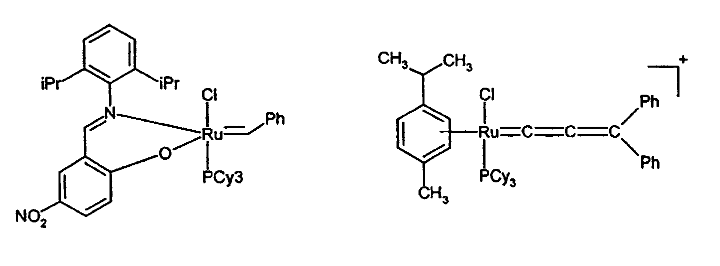 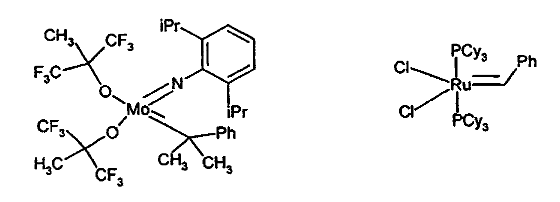 wobei Cy = Cyclohexyl, iPr = Isopropyl, Ph = Phenyl bedeutet

Die Katalysatoren können in isolierter Form eingesetzt oder in situ im Reaktionsmedium aus Katalysatorvorstufen erzeugt werden. Die Katalysatormengen, die für das erfindungsgemäße Verfahren eingesetzt werden, liegen in der Regel bei 0,001 bis 15 mol%, bezogen auf die Edukte. Bevorzugt wird das erfindungsgemäße Verfahren mit 0,1 bis 12 mol% Katalysator, besonders bevorzugt mit 0,5 bis 9 mol% bezogen auf die Edukte, durchgeführt.

Die für das erfindungsgemäße Verfahren eingesetzten ionischen Flüssigkeiten sind Salze oder Salzmischungen, die in einem Temperaturintervall von -20°C bis 300°C flüssig sind.

Bevorzugt werden für das erfindungsgemäße Verfahren ionische Flüssigkeiten eingesetzt, die Aluminiumhalogenide in Kombination mit mindestens einem quartären Ammoniumhalogenid und/oder mindestens einem quartärem Phosphoniumhalogenid enthalten.

Besonders bevorzugt werden als quartäre Ammoniumverbindungen heterocyclische Verbindungen, die mindestens ein Stickstoffatom enthalten, verwendet. Dies sind beispielsweise Pyridiniumverbindungen oder Imidazoliumverbindungen

Ganz besonders bevorzugt werden als ionische Flüssigkeiten Aluminiumchlorid in Kombination mit 1-Methyl-3-butylimidazoliumchlorid, 1-Methyl-3-ethylimidazoliumchlorid, N-Butylpyridiniumchlorid und/oder Tetrabutylphosphoniumchlorid verwendet.

Bevorzugt werden für das erfindungsgemäße Verfahren ionische Flüssigkeiten eingesetzt, die aus einer Kombination von Aluminiumhalogenid und quartären Ammoniumhalogeniden und/oder quartären Phosphoniumhalogeniden im molaren Verhältnis (0,6 - 1) : 1 bestehen. Das molare Verhältnis von Aluminiumhalogenid zu quartärem Ammoniumhalogenid und/oder quartärem Phosphoniumhalogenid sollte nie größer als 1 sein, da sonst ein Überschuß an Lewis-Säure in der Reaktionsmischung vorliegt. Diese saure Reaktionsmischung führt zu einer Desaktivierung der Übergangsmetallcarben-Katalysatoren.

Weiterhin bevorzugt werden für das erfindungsgemäße Verfahren ionische Flüssigkeiten eingesetzt, die Ammoniumhexafluorophosphat, Ammoniumtetrafluoroborat, Ammoniumtosylat, oder Ammoniumhydrogensulfat enthalten oder aus Ammoniumhexafluorophosphat, Ammoniumtetrafluoroborat, Ammoniumtosylat oder Ammoniumhydrogensulfat bestehen.

Besonders bevorzugt werden als ionische Flüssigkeiten Pyridiniumhexafluorophosphat, 1-Methyl-3-butylhexafluorophosphat, Pyridiniumtetrafluoroborat, Pyridiniumhydrogensulfat oder N-Butylpyridiniumhexafluorophosphat eingesetzt.

Im erfindungsgemäßen Verfahren können als ionische Flüssigkeiten auch Kombinationen von Aluminiumhalogenid mit Mischungen von quartären Ammoniumhalogeniden und/oder quartären Phosphoniumhalogeniden sowie Mischungen von Ammoniumhexafluorophosphaten, Ammoniumtetrafluoroboraten, Ammoniumtosylaten oder Ammoniumhydrogensulfaten eingesetzt werden.

Das erfindungsgemäße Verfahren kann in Gegenwart von einem oder mehreren Additiven, bevorzugt eines Lösungsmittels, durchgeführt werden, wodurch beispielsweise eine leichtere Abtrennung der Produkte von der ionischen Flüssigkeit und des darin befindlichen Katalysators möglich wird. In Gegenwart eines Additivs und der ionischen Flüssigkeiten ergibt sich dann ein heterogenes Katalysesystem, wenn ionische Katalysatoren oder Katalysatoren, die sich bevorzugt in der ionischen Flüssigkeit lösen, eingesetzt werden. Nach Beendigung der Metathesereaktion kann die ionische Phase, die den Katalysator enthält, einfach vom Additiv mit dem darin befindlichen Reaktionsprodukt abgetrennt werden. Der Katalysator in der ionischen Flüssigkeit kann ohne Reinigungszwischenschritte für weitere Metathesereaktionen eingesetzt werden.

Solche Additive können beispielsweise gewählt werden aus: Phosphorverbindungen, Amine, perfluorierte Verbindungen, Metallalkoxide und organische Lösungsmittel. Als organische Lösungsmittel eignen sich insbesondere halogenierte Kohlenwasserstoffe wie beispielsweise Dichlormethan, Trichlormethan, Tetrachlormethan, 1,2 Dichlorethan, Trichlorethan, aromatische Verbindungen wie beispielsweise Benzol, Toluol, Xylol, Cumol, Halogenbenzole, Alkane wie beispielsweise Pentan, Hexan, Cyclohexan, Ester wie beispielsweise tert.-Butylmethylester oder Acetessigester, Ether wie beispielsweise Diethylether, Tetrahydrofuran, Dimethoxyethan, Amide wie beispielsweise Dimethylformamid, Antioxidantien wie beispielsweise Hydrochinone, Aceton, Dimethylcarbonat oder Alkohole.

Bevorzugt werden als Additive C₅-C₂₀-Alkane, Ether oder halogenierte Kohlenwasserstoffe für das erfindungsgemäße Verfahren eingesetzt.

Ganz besonders bevorzugt werden im erfindungsgemäßen Verfahren Pentan, n-Hexan, Methyl-*tert.*-butylether oder Dichlormethan eingesetzt.

Bevorzugt wird das erfindungsgemäße Verfahren bei Drücken im Bereich von 0,1 bis 10 bar durchgeführt, insbesondere bevorzugt bei Atmosphärendruck. Das erfindungsgemäße Verfahren kann aber auch bei Unterdrücken bis 0,01 bar und Überdrücken bis 100 bar durchgeführt werden.

Üblicherweise wird das erfindungsgemäße Verfahren in einem Temperaturbereich von -20°C bis 200 °C durchgeführt, bevorzugt von 0°C bis 150°C, ganz besonders bevorzugt von 20°C bis 100°C.

Die nach dem erfindungsgemäßen Verfahren herstellbaren cyclischen Verbindungen oder Polymere können mit Hilfe gängiger Methoden weiter gereinigt und verarbeitet werden. Der Katalysator in der ionischen Flüssigkeit kann ohne Reinigungszwischenschritte für weitere Metathesereaktionen eingesetzt werden.

### Beispiele

Die im folgenden angeführten Beispiele beschreiben Metathesereaktionen in Gegenwart von ionischen Flüssigkeiten (und Additiven) unter bevorzugten Bedingungen. Sie sollen jedoch in keiner Weise den Umfang der vorliegenden Erfindung einschränken. Die Abkürzung Cy steht für Cyclohexyl, Ph für Phenyl, iPr für Isopropyl und TfO für Triflat.

### Beispiel 1

### Herstellung von N-Carboxymethyl-2,5-dihydropyrrol

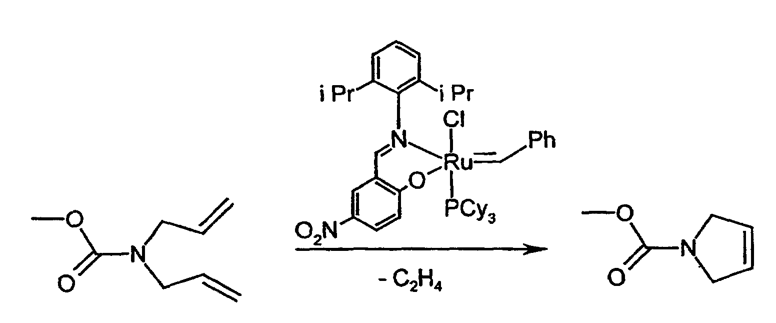

155 mg (1 mmol) N-Carboxymethyl-diallylamin und 17 mg (Tricyclohexylphosphin)-benzyliden-chloro-ruthenium(IV)-2-[(2,6-diisopropylphenyl)imino]methyl4-nitro-phenolat (2 mol%) wurden in einer flüssigen Mischung von 579 mg 1-Methyl-3-ethylimidazoliumchlorid (4 mmol) und 533 mg Aluminiumtrichlorid (4 mmol) unter Argonatmosphäre gelöst. Die Mischung wurde mit 3 ml absolutem n-Hexan überschichtet. Man ließ 30 min unter Rühren bei Raumtemperatur reagieren. Nach Phasentrennung wurde die ionische Phase noch drei mal mit n-Hexan gewaschen. Eine gaschromatographische Untersuchung der organischen Phase ergab einen Produktanteil von 30% bezüglich N-Carboxymethyl-2,5-dihydropyrrol. Ein erneutes Überschichten der Katalysatorphase mit einer Lösung von 155 mg (1 mmol) N-Carboxymethyl-diallylamin in 3 ml n-Hexan, erneuter 30 minütiger Reaktionszeit und einer analogen Aufarbeitung ergab eine Ausbeute von 20% bezüglich N-Carboxymethyl-2,5-dihydropyrrol.

### Beispiel 2

### Herstellung von N-Carboxymethyl-3,4,5,6-tetrahydroanilin

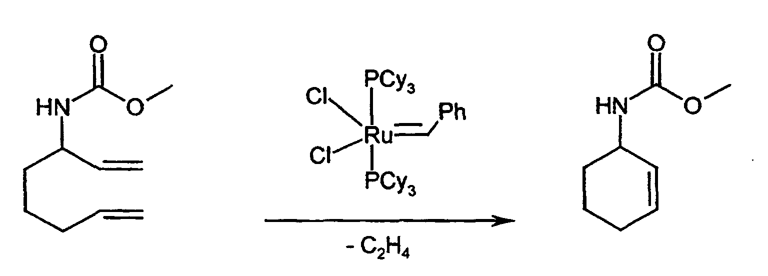

92 mg (0.5 mmol) N-Carboxymethyl-3-amino-1,7-octadien und 4 mg Bis(tricyclohexylphosphin)-benzyliden-ruthenium(IV)-dichlorid (1 mol%) wurden in einem ausgeheizten Schlenkrohr unter Argonatmosphäre in einer flüssigen Mischung von 586 mg 1-Methyl-3-ethylimidazoliumchlorid (4 mmol) und 533 mg Aluminiumtrichlorid (4 mmol) gelöst. Man ließ zwei Stunden bei 50°C reagieren. Nach wäßriger Aufarbeitung wurde über eine sehr kurze Kieselgelsäule (0,5 cm) filtriert, viermal mit je 1 ml Acetonitril nachgewaschen und eingeengt.
Ausbeute: 77 mg N-Carboxymethyl-3,4,5,6-tetrahydroanilin (0.49 mmol, 99% der Theorie).
¹H NMR (400 MHz, CDCl₃) δ 5.85 (1H, d, *J*= 9.0 Hz), 5.60 (1H, d, *J*= 9.0 Hz), 4.70 (1H, s), 4.20 (1H, s), 3.65 (3H, s), 1.98 (2H, m), 1.90 (1H, m), 1.62 (2H, m), 1.52 (1H, m).

### Beispiel 3

### Herstellung von N-Carboxymethyl-3,4,5,6-tetrahydroanilin

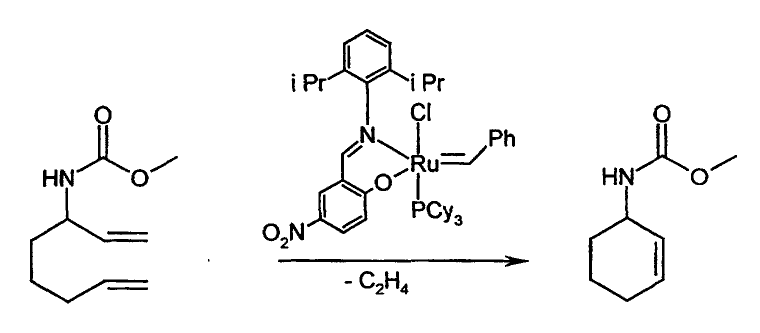

92 mg (0.5 mmol) N-Carboxymethyl-3-amino-1,7-octadien und 10 mg des in dem obigen Reaktionsschema gezeigten Rutheniumkatalysators (Tricyclohexylphosphin)-benzyliden-chloro- ruthenium-(IV)-2-[(2,6-diisopropylphenyl)imino]methyl-4-nitrophenolat wurden in einem ausgeheizten Schlenkrohr unter Argonatmosphäre in 1 ml abs. Hexan und einem Gemisch von 290 mg (2 mmol) 1-Methyl-3-ethylimidazoliumchlorid und 266 mg (2 mmol) Aluminiumtrichlorid gelöst. Man ließ 3 h bei 50°C reagieren. Zur Aufarbeitung wurde die organische Phase abpipettiert und die Phase der ionischen Flüssigkeiten noch zwei mal mit je 2 ml Hexan gewaschen. Die vereinigten Hexanphasen wurden eingeengt Abschließend wurde über eine sehr kurze Kieselgelsäule (0,2 cm) filtriert, einmal mit 1 ml Acetonitril nachgewaschen und eingeengt. Der in der ionischen Flüssigkeit gelöste Katalysator steht für eine weitere Umsetzung mit Substrat in Hexan zur Verfügung.
Ausbeute: 74 mg N-Carboxymethyl-3,4,5,6-tetrahydroanilin (0.48 mmol, 96% der Theorie).

### Beispiel 4

### Herstellung von N-Carboxymethyl-3,4,5,6-tetrahydroanilin

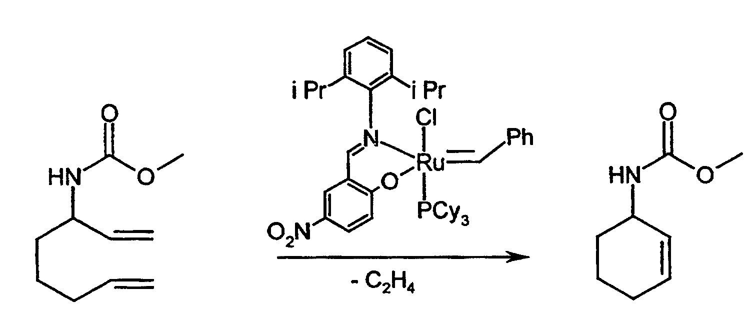

92 mg (0.5 mmol) N-Carboxymethyl-3-amino-1,7-octadien und 4 mg Bis(tricyclohexylphosphin)benzylidenruthenium(IV)dichlorid (1 mol%) wurden in einem ausgeheizten Schlenkrohr unter Argonatmosphäre in einer flüssigen Mischung von 684 mg N-Butylpyridiniumchlorid (4 mmol) und 533 mg Aluminiumtrichlorid (4 mmol) gelöst. Man ließ zwei Stunden bei 50°C reagieren. Nach wäßriger Aufarbeitung wurde über eine sehr kurze Kieselgelsäule (0,5 cm) filtriert, viermal mit je 1 ml Acetonitril nachgewaschen und eingeengt. Ausbeute: 77 mg N-Carboxymethyl-3,4,5,6-tetrahydroanilin (0.49 mmol, 98% der Theorie).

### Beispiel 5

### Herstellung von Tetrahydrobenzol

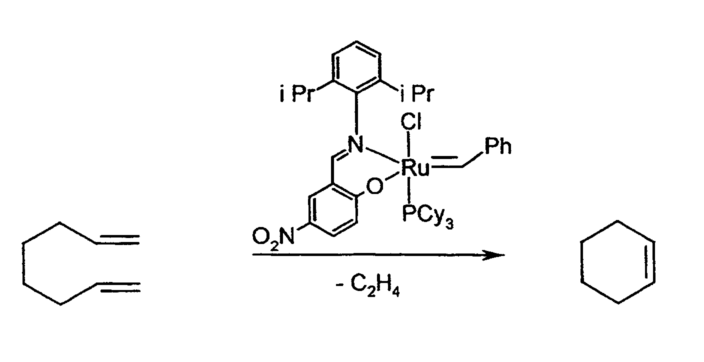

110 mg (1 mmol) 1,7-Octadien und 17 mg (Tricyclohexylphosphin)-benzylidenchloro-ruthenium-(IV)-2-[(2,6-diisopropylphenyl)imino]methyl-4-nitro-phenolat (2 mol%) wurden in einem ausgeheizten Schlenkrohr unter Argonatmosphäre in 3 ml absolutem Pentan gelöst. Es wurde eine flüssige Mischung von 579 mg 1-Methyl-3-ethylimidazoliumchlorid (4 mmol) und 533 mg Aluminiumtrichlorid (4 mmol) hinzugegeben. Man ließ 30 Minuten bei Raumtemperatur reagieren. Zur Aufarbeitung wurde die organische Phase abpipettiert und die Phase der ionischen Flüssigkeiten noch zwei mal mit je 2 ml Pentan gewaschen. Die vereinigten Pentanphasen wurden eingeengt Abschließend wurde über eine sehr kurze Kieselgelsäule (0,2 cm) filtriert, einmal mit 1 ml Pentan nachgewaschen und eingeengt. Die Ausbeute an Tetrahydrobenzol wurde durch Gaschromatographie bestimmt (interner Standard: Tetradecan) und betrug 99% der Theorie.

Nach erneutem Überschichten der Reaktionslösung mit einer Mischung von 110 mg (1 mmol) 1,7-Octadien in Pentan, einer Reaktionszeit von 30 Minuten und anschließender Phasentrennung erhielt man eine Ausbeute von 63% Tetrahydrobenzol.

### Beispiel 6

### Herstellung von Tetrahydrobenzol

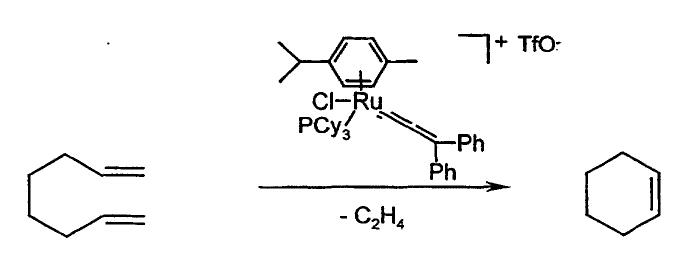

28 mg 1,7-Octadien (0.25 mmol) wurden unter Argonatmosphäre in einem 20 ml Schlenkrohr in einer Mischung von 345 mg (2 mmol) 1-Methyl-3-butylimidazoliumchlorid und 266 mg (2 mmol) Aluminiumtrichlorid gelöst Es wurden 11 mg des im obigen Reaktionsschema beschriebenen kationischen Katalysators hinzugegeben (5 mol%). Die Reaktionslösung wurde mit 3 ml Methyl-*tert*.-butylether überschichtet. Man ließ 90 Minuten bei einer Temperatur von 40°C reagieren und trennte danach die organische Phase ab. Die ionische Phase wurde noch dreimal mit Methyl*tert*.-butylether extrahiert. Gaschromatographisch wurde ein Gehalt von 32% an gewünschtem Tetrahydrobenzol ermittelt. Dieses Ergebnis wurde in einer anschließenden zweiten Metathesereaktion unter Verwendung derselben ionischen Phase wieder erreicht.

### Beispiel 7

### Herstellung von Aminocyclotridec-11-en-2-on

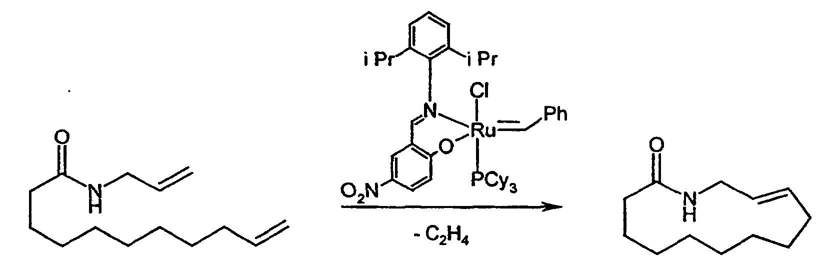

58 mg (0.25 mmol) 10-Undecenoyl-allylamid und 10 mg (Tricyclohexylphosphin)-benzyliden-chloro-ruthenium-(IV)-2-[(2,6-diisopropylphenyl)imino]methyl-4-mtrophenolat (5 mol%) wurden in 3 g 1-Methyl-3-butylimidazoliumhexafluorophosphat gelöst. Man ließ bei 40°C 14 h reagieren. Die ionische Phase wurde mit Dichlormethan extrahiert. Nach Filtration über eine sehr kurze Kieselgelsäule (Länge ca. 0.5 cm) wurden ca. 80% des gewünschten Produktes erhalten (Ausbeutebestimmung mittels Gaschromatographie). Das E/Z-Verhältnis beträgt hierbei ca. 5:1.

### Beispiel 8

### Metathese von 10-Undecensäure-buten-4-yl-ester

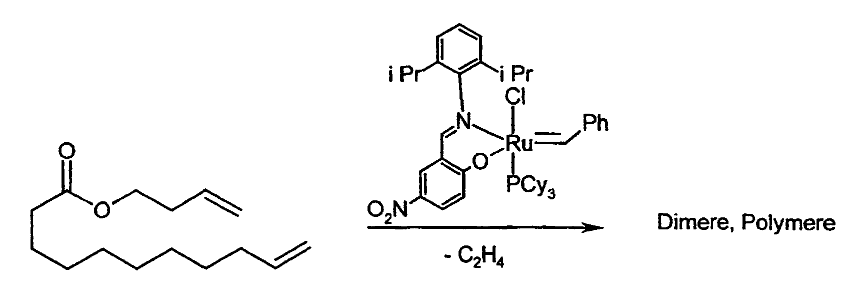

238 mg 10-Undecensäure-buten-4-yl-ester und 8 mg (Tricyclohexylphosphin)-benzyliden-chloro-ruthenium-(IV)-2-[(2,6-diisopropylphenyl)imino]methyl-4-nitrophenolat (8 mol%) wurden in einer Mischung von 580 mg (4 mmol) 1-Methyl-3-ethylimidazoliumchlorid und 533 mg Aluminiumtrichlorid (4 mmol) gelöst. Die Mischung wurde mit 40 ml absolutem Pentan überschichtet. Man ließ unter guter Durchmischung über einen Zeitraum von fünf Stunden bei Raumtemperatur reagieren. Es wurden neben einem großen Prozentsatz Polymer ca. 10 % Dimere des eingesetzten Diens erhalten.

### Beispiel 9

### Herstellung von 3,3'-Bis-2,5-dihydrofuranyl

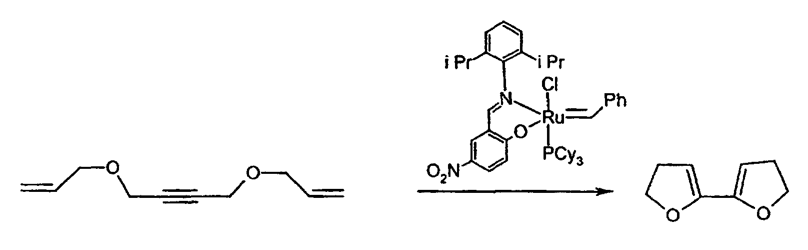

166 mg 1,4-Bis-oxypropen-2-yl-butin-2 wurden mit 41 mg (Tricyclohexylphosphin)-benzyliden-chloro-ruthenium-(IV)-2-[(2,6-diisopropylphenyl)imino]methyl-4-nitrophenolat (5 mol%) in einem ausgeheizten Schlenkrohr unter Argonatmosphäre in einer flüssigen Mischung von 2,31 g 1-Methyl-3-ethylimidazoliumchlorid (16 mmol) und 2,13 g Aluminiumtrichlorid (16 mmol) gelöst Man ließ 18 Stunden bei 40°C reagieren. Zur Aufarbeitung wurde die ionische Phase mehrfach mit Toluol gewaschen. Die vereinigten Toluolextrakte wurden über eine sehr kurze Kieselgelsäule (0,5 cm) filtriert. Es wurde viermal mit je 10 ml Toluol nachgewaschen und eingeengt.
Ausbeute: 120 mg 3,3'-Bis-2,5-dihydrofuranyl (87% der Theorie).

## Patentansprüche

1. Verfahren zur Herstellung cyclischer und/oder polymerer Verbindungen durch Metathese von Edukten, die mindestens zwei funktionelle Gruppen in Form von substituierten oder unsubstituierten Alken- oder Alkineinheiten enthalten, in Gegenwart von einem oder mehreren homogen oder heterogen vorliegenden Katalysatoren, **dadurch gekennzeichnet, daß** die Metathese in Gegenwart von ionischen Flüssigkeiten durchgeführt wird und daß als Katalysatoren Übergangsmetallcarbene oder Übergangsmetallverbindungen, die unter den Reaktionsbedingungen Übergangsmetallcarbene bilden oder Übergangsmetallsalze in Verbindung mit einem Alkylierungsmittel verwendet werden.

2. Verfahren nach Anspruch 1, **dadurch gekennzeichnet, daß** es sich bei den polymeren Verbindungen um Homopolymere, Copolymeren oder Block-Copolymere handelt.

3. Verfahren nach Anspruch 1, **dadurch gekennzeichnet, daß** es sich bei den cyclischen Verbindungen um carbo- oder heterocyclische Verbindungen mit Ringgrößen ≥ 5 Ringgliedern handelt.

4. Verfahren gemäß einem oder mehreren der vorangegangenen Ansprüche 1 bis 3, **dadurch gekennzeichnet, daß** die Edukte neben den an der Metathesereaktion beteiligten funktionellen Gruppen mindestens einen weiteren, sich in der Metathesereaktion inert verhaltenden Substituenten und/oder ein Heteroatom enthalten.

5. Verfahren nach Anpruch 4, **dadurch gekennzeichnet, daß** die genannten Substituenten oder Heteroatome unabhängig gewählt werden aus: verzweigte oder unverzweigte Alkylreste, aromatische oder nicht-aromatische carbocyclische Ringe, Carbonsäuren, Ester, Ether, Epoxide, Silylether, Thioether, Thioacetale, Anhydride, Imine, Silylenolether, Ammoniumsalze, Amide, Nitrile, Perfluoralkyl-Gruppen, gem.-Dialkyl-Gruppen, Alkine, Alkene, Halogene, Alkohole, Ketone, Aldehyde, Carbamate, Carbonate, Urethane, Sulfonate, Sulfone, Sulfonamide, Nitro-Gruppen, Organosilan-Einheiten, Metallzentren, Sauerstoff-, Stickstoff-, schwefel-, phosphorhaltige Heterocyclen.

6. Verfahren gemäß Anspruch 5, **dadurch gekennzeichnet, daß** als Edukte α,ω-Diene eingesetzt werden, die mindestens einen weiteren, sich in der Metathesereaktion inert verhaltenden Substituenten und/oder ein Heteroatom enthalten können, wobei diese Substituenten oder Heteroatome unabhängig gewählt werden können aus verzweigte oder unverzweigte Alkylreste, aromatische oder nicht-aromatische carbocyclische Ringe, Carbonsäuren, Ester, Ether, Epoxide, Silylether, Thioether, Thioacetale, Anhydride, Imine, Silylenolether, Ammoniumsalze, Amide, Nitrile, Perfluoralkyl-Gruppen, gem.-Dialkyl-Gruppen, Alkine, Alkene, Halogene, Alkohole, Ketone, Aldehyde, Carbamate, Carbonate, Urethane, Sulfonate, Sulfone, Sulfonamide, Nitro-Gruppen, Organosilan-Einheiten, Metallzentren, sauerstoff-, Stickstoff-, schwefel-, phosphorhaltige Heterocyclen.

7. Verfahren gemäß Anspruch 6, **dadurch gekennzeichnet, daß** α,ω-Diene, die in α-Stellung zu einer Doppelbindung einen Substituenten NRR' tragen, eingesetzt werden, wobei
R ein organischer Substituent, bevorzugt Wasserstoff, gegebenenfalls anelliertes Aryl, Alkyl, CN, COOR² oder Halogen ist,
R¹ *tert*.-Butyl, P(R)₂, P(R²)₂, COR, SO₂PhR, COOR oder CONRR² ist,
R² Alkyl oder Phenyl ist,
R und R¹ gemeinsam 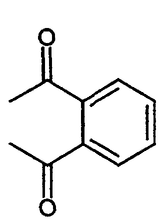 bedeuten
und besagte α,ω-Diene auch an jeder anderen Position im Molekül, mit Ausnahme der α-Stellung, mindestens einen weiteren Substituenten R tragen können.

8. Verfahren gemäß Anspruch 7, **dadurch gekennzeichnet, daß** α,ω-Diene der allgemeinen Formel (I) eingesetzt werden 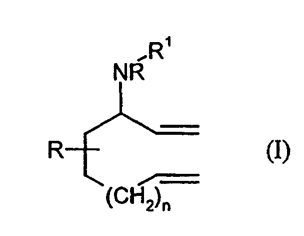 in welcher R, R¹ und R² die in Anspruch 7 genannte Bedeutung haben und n die Zahl 1,2,3 oder 4, bevorzugt 1 oder 2, besonders bevorzugt 1 bedeutet.

9. Verfahren gemäß Anspruch 7, **dadurch gekennzeichnet, daß** als α,ω-Diene Diallylamin oder 3-Amino-1,7-octadien, besonders bevorzugt in ihrer N-Carboxymethyl-geschützten Form oder 1,7-Octadien, 10-Undecenoyl-allylamid, 1,4-Bis-oxypropen-2-yl-butin-2 oder 10-Undecensäure-buten-4-yl-ester eingesetzt werden.

10. Verfahren gemäß einem oder mehreren der vorangegangenen Ansprüche 1 bis 9, **dadurch gekennzeichnet, daß** als Katalysatoren Verbindungen der allgemeinen Formel (III) bis (VI) eingesetzt werden können, wobei M Ruthenium oder Osmium bedeutet 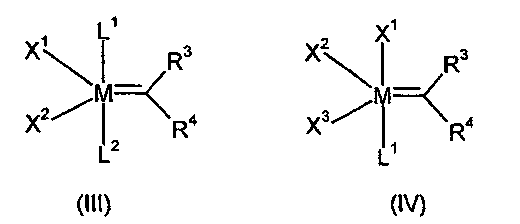 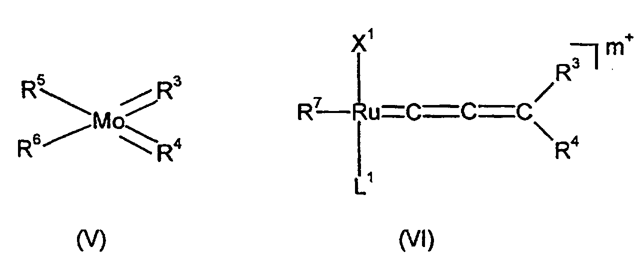 und
R³ bis R⁷ unabhängig voneinander wählbare Reste sind aus: Wasserstoff, C₁-C₂₀ Alkyl, C₃-C₈ Cycloalkyl, C₂-C₂₀ Alkenyl, C₂-C₂₀ Alkinyl, C₆-C₁₈ Aryl, C₁-C₂₀ Carboxylat, C₁-C₂₀ Alkoxy, C₂-C₂₀ Alkenyloxy, C₂-C₂₀ Alkinyloxy, C₆-C₁₈ Aryloxy, C₂-C₂₀ Alkoxycarbonyl, C₁-C₂₀ Alkylthio, C₁-C₂₀ Alkylsulfonyl oder C₁-C₂₀ Alkylsulfinyl, N-Aryl; jeweils wahlweise substituiert mit C₁-C₁₂ Alkyl, Perfluoralkyl, Halogen, C₁-C₅ Alkoxy oder C₆-C₁₈ Aryl, wobei R³ bis R⁷ auch in cyclischen Verbindungen miteinander verknüpft vorliegen können,
X¹ bis X³ unabhängig voneinander wählbare anionische Liganden sind, insbesondere F⁻, Cl⁻, Br⁻, CN⁻, SCN⁻,R³O⁻, R³R⁴N⁻, (R³-R⁷)-Allyl⁻, (R³-R⁷)-Cyclopentadienyl⁻, wobei die Reste R³ bis R⁷ die bereits genannte Definition erfüllen,
L¹ bis L³ unabhängig voneinander wählbare neutrale Liganden sind, insbesondere CO, CO₂, R³NCO, R³R⁴C=CR⁵R⁶, R³C≡CR⁴, R³R⁴C=NR⁵, R³C≡N, R³OR⁴, R³SR⁴, NR³R⁴R⁵, PR³R⁴R⁵, AsR³R⁴R⁵, SbR³R⁴R⁵, wobei die Reste R³ bis R⁵ die bereits genannte Definition erfüllen
und
m 1 oder 2 ist.

11. Verfahren nach Anspruch 10, **dadurch gekennzeichnet, daß** als Katalysatoren oder Katalysatorvorstufen Verbindungen der allgemeinen Formel (III) und/oder (IV) mit L¹ und L² = PR³R⁴R⁵ verwendet werden, wobei R³ bis R⁵ den oben genannten Definitionen entsprechen, ganz besonders bevorzugte Reste sind dabei Aryl oder Alkyl, insbesondere sekundäre Alkylreste oder Cycloalkylreste.

12. Verfahren nach Anspruch 11, **dadurch gekennzeichnet, daß** als Katalysatoren folgende Verbindungen eingesetzt werden:

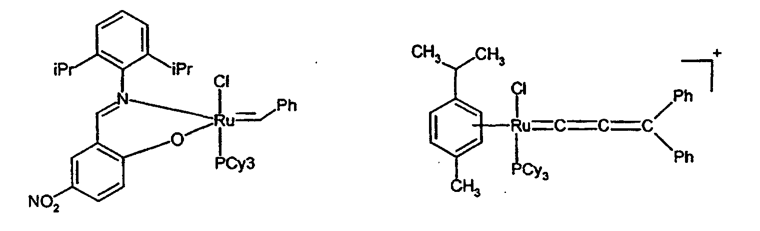

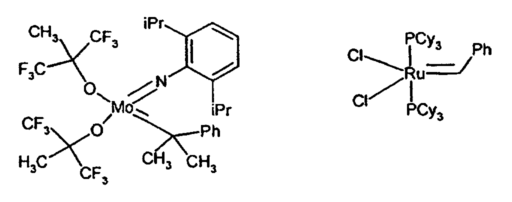

13. Verfahren nach einem oder mehreren der vorangegangenen Ansprüchen 1 bis 12, **dadurch gekennzeichnet, daß** als ionische Flüssigkeiten Ammoniumhexafluorophosphat, Ammoniumtetrafluoroborat, Ammoniumtosylat oder Ammoniumhydrogensulfat eingesetzt werden oder Salzmischungen eingesetzt werden, die Aluminiumhalogenide in Kombination mit mindestens einem quartären Ammoniumhalogenid und/oder mindestens einem quartärem Phosphoniumhalogenid enthalten.

14. Verfahren nach Anspruch 13, **dadurch gekennzeichnet, daß** als ionische Flüssigkeiten Pyridiniumhexafluorophosphat, Pyridiniumtetrafluoroborat, Pyridiniumhydrogensulfat, 1-Methyl-3-butylimidazoliumhexafluorophosphat oder Kombinationen von Aluminiumchlorid mit 1-Methyl-3-butylimidazoliumchlorid, 1-Methyl-3-ethylimidazoliumchlorid, N-Butylpyridiniumchlorid und/oder Tetrabutylphosphoniumhalogenid verwendet werden.

15. Verfahren nach einem oder mehreren der vorangegangenen Ansprüche 1 bis 14, **dadurch gekennzeichnet, daß** als ionische Flüssigkeiten auch Kombinationen von Aluminiumhalogenid mit Mischungen von quartären Ammoniumhalogeniden und/oder quartären Phosphoniumhalogeniden, sowie Mischungen von Ammoniumhexafluorophosphat, Ammoniumtetrafluoroborat, Ammoniumtosylat oder Ammoniumhydrogensulfat eingesetzt werden können.

16. Verfahren nach einem oder mehreren der vorangegangenen Ansprüche 1 bis 15, **dadurch gekennzeichnet, daß** das Reaktionsmedium zusätzlich ein oder mehrere Additive enthält, die unabhängig gewählt werden aus: Phosphorverbindungen, Amine, perfluorierte Verbindungen, Metallalkoxide oder organischen Lösungsmitteln.

## Claims

1. Process for preparing cyclic and/or polymeric compounds by metathesis of starting materials which contain at least two functional groups in the form of substituted or unsubstituted alkene or alkine units in the presence of one or more homogeneous or heterogeneous catalysts, **characterized in that** the metathesis is carried out in the presence of ionic liquids and the catalysts used are transition metal carbenes or transition metal compounds which form transition metal carbenes under the reaction conditions or transition metal salts in combination with an alkylating agent.

2. Process according to Claim 1, **characterized in that** the polymeric compounds are homopolymers, copolymers or block copolymers.

3. Process according to Claim 1, **characterized in that** the cyclic compounds are carbocyclic or heterocyclic compounds having ring sizes of ≥ 5 ring atoms.

4. Process according to one or more of Claims 1 to 3, **characterized in that** the starting materials contain, apart from the functional groups participating in the metathesis reaction, at least one further substituent which is inert in the metathesis reaction and/or a heteroatom.

5. Process according to Claim 4, **characterized in that** the substituents or heteroatoms mentioned are selected independently from among: branched or unbranched alkyl radicals, aromatic or non-aromatic carbocyclic rings, carboxylic acids, esters, ethers, epoxides, silyl ethers, thioethers, thioacetals, anhydrides, imines, silylenol ethers, ammonium salts, amides, nitriles, perfluoroalkyl groups, geminal dialkyl groups, alkines, alkenes, halogens, alcohols, ketones, aldehydes, carbamates, carbonates, urethanes, sulphonates, sulphones, sulphonamides, nitro groups, organosilane units, metal centres and oxygen-, nitrogen-, sulphur- and/or phosphorus-containing heterocycles.

6. Process according to Claim 5, **characterized in that** the starting materials used are α,ω-dienes which may contain at least one further substituent which is inert in the metathesis reaction and/or a heteroatom, where these substituents or heteroatoms may be selected independently from among branched or unbranched alkyl radicals, aromatic or non-aromatic carbocyclic rings, carboxylic acids, esters, ethers, epoxides, silyl ethers, thioethers, thioacetals, anhydrides, imines, silylenol ethers, ammonium salts, amides, nitriles, perfluoroalkyl groups, geminal dialkyl groups, alkines, alkenes, halogens, alcohols, ketones, aldehydes, carbamates, carbonates, urethanes, sulphonates, sulphones, sulphonamides, nitro groups, organosilane units, metal centres and oxygen-, nitrogen-, sulphur- and/or phosphorus-containing heterocycles.

7. Process according to Claim 6, **characterized in that** the α,ω-dienes used bear a substituent NRR¹ in the α position to a double bond, where
R is an organic substituent, preferably hydrogen, fused or unfused aryl, alkyl, CN, COOR² or halogen,
R¹ is tert-butyl, P(R)₂, P(R²)₂, COR, SO₂PhR, COOR or CONRR²,
R² is alkyl or phenyl,
R and R¹ together form 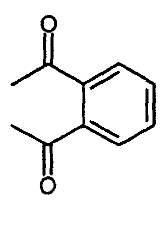 and
said α,ω-dienes may also bear at least one further substituent R in any other position in the molecule with the exception of the α position.

8. Process according to Claim 7, **characterized in that** the α,ω-dienes used have the formula (I) 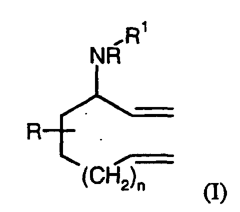 where R, R¹ and R² are as defined in Claim 7 and
n is 1, 2, 3 or 4, preferably 1 or 2, particularly preferably 1.

9. Process according to Claim 7, **characterized in that** the α,ω-dienes used are diallylamine or 3-amino-1,7-octadiene, particularly preferably in their N-carboxymethyl-protected form, or 1,7-octadiene, 10-undecenoyl-allylamide, 1,4-bis-oxypropen-2-yl-but-2-ine or buten-4-yl 10-undecenoate.

10. Process according to one or more of Claims 1 to 3, wherein the catalysts used are compounds of the formulae (III) to (VI), where M is ruthenium or osmium 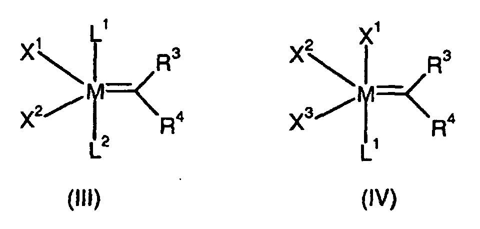 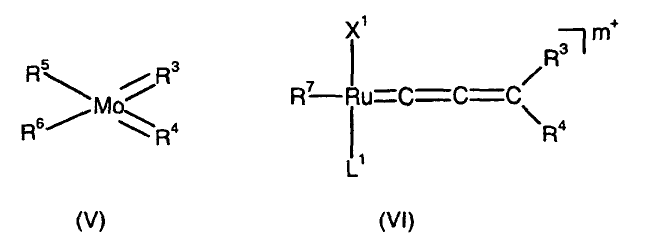 and
R³ to R⁷ are radicals which can be selected independently from among hydrogen, C₁-C₂₀-alkyl, C₃-C₈-cycloalkyl, C₂-C₂₀-alkenyl, C₂-C₂₀-alkinyl, C₆-C₁₈-aryl, C₁-C₂₀-carboxylate, C₁-C₂₀-alkoxy, C₂-C₂₀-alkenyloxy, C₂-C₂₀-alkinyloxy, C₆-C₁₈-aryloxy, C₂-C₂₀-alkoxycarbonyl, C₁-C₂₀-alkylthio, C₁-C₂₀-alkylsulphonyl or C₁-C₂₀-alkylsulphinyl, N-aryl; in each case unsubstituted or substituted by C₁-C₁₂-alkyl, perfluoroalkyl, halogen, C₁-C₅-alkoxy or C₆-C₁₈-aryl. The radicals R³ to R⁷ may be linked to one another in cyclic compounds,
X¹ to X³ are anionic ligands which may be selected independently, in particular F, Cl⁻, Br⁻, CN⁻, SCN⁻, R³O⁻, R³R⁴N⁻, (R³-R⁷)-allyl⁻, (R³-R⁷)-cyclopentadienyl⁻, where the radicals R³ to R⁷ are as defined above,
L¹ to L³ are uncharged ligands which can be selected independently, in particular CO, CO₂, R³NCO, R³R⁴C=CR⁵R⁶, R³C≡R⁴, R³R⁴C=NR⁵, R³C≡N, R³OR⁴, R³SR⁴, NR³R⁴R⁵, PR³R⁴R⁵, AsR³R⁴R⁵, SbR³R⁴R⁵, where the radicals R³ to R⁵ are as defined above
and
m is 1 or 2.

11. Process according to Claim 10, **characterized in that** the catalysts or catalyst precursors used are compounds of the formula (III) and/or (IV) in which L¹ and L² = PR³R⁴R⁵, where R³ to R⁵ are as defined above and very particularly preferred radicals of this type and aryl or alkyl, in particular secondary alkyl radicals or cycloalkyl radicals.

12. Process according to Claim 11, **characterized in that** the catalysts used are the following compounds:

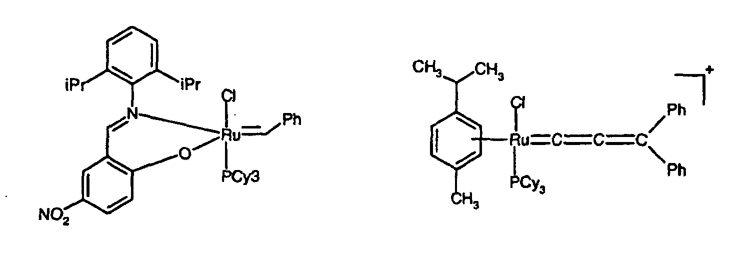

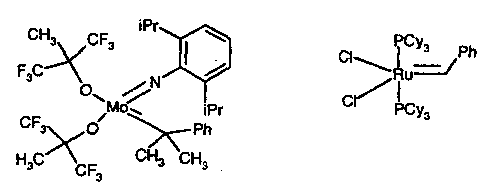

13. Process according to one or more of Claims 1 to 12, **characterized in that** the ionic liquids used are ammonium hexafluorophosphate, ammonium tetrafluoroborate, ammonium tosylate or ammonium hydrogen sulphate or salt mixtures comprising aluminium halides in combination with at least one quaternary ammonium halide and/or at least one quaternary phosphonium halide.

14. Process according to Claim 13, **characterized in that** the ionic liquids used are pyridinium hexafluorophosphate, pyridinium tetrafluoroborate, pyridinium hydrogen sulphate, 1-methyl-3-butylimidazolium hexafluorophosphate or combinations of aluminium chloride with 1-methyl-3-butylimidazolium chloride, 1-methyl-3-ethylimidazolium chloride, N-butylpyridinium chloride and/or tetrabutylphosphonium halide.

15. Process according to one or more of Claims 1 to 14, **characterized in that** the ionic liquids used are combinations of aluminium halide with mixtures of quaternary ammonium halides and/or quaternary phosphonium halides, or mixtures of ammonium hexafluorophosphate, ammonium tetrafluoroborate, ammonium tosylate or ammonium hydrogen sulphate.

16. Process according to one of more of Claims 1 to 15, **characterized in that** the reaction medium further comprises one or more additives which are selected independently from among: phosphorus compounds, amines, perfluorinated compounds, metal alkoxides or organic solvents.

## Revendications

1. Procédé de préparation de composés cycliques et/ou de polymères par métathèse d'adducts, qui contiennent au moins deux groupes fonctionnels sous forme d'unités alcènes ou alcynes substituées ou non substituées, en présence d'un ou de plusieurs catalyseurs présents de manière homogène ou hétérogène, **caractérisé en ce qu'**on effectue la métathèse en présence de liquides ioniques et qu'on utilise comme catalyseurs des carbènes de métaux de transition ou des composés de métaux de transition qui forment des carbènes de métaux de transition dans les conditions réactionnelles ou des sels de métaux de transition en liaison avec un agent d'alkylation.

2. Procédé selon la revendication 1, **caractérisé en ce que** comme composés polymères il s'agit d'homopolymères, de copolymères ou de copolymères séquencés.

3. Procédé selon la revendication 1, **caractérisé en ce que** comme composés cycliques il s'agit de composés carbo- ou hétérocycliques avec une grosseur de cycle ≥ 5 membres cycliques.

4. Procédé selon une ou plusieurs des revendications précédentes 1 à 3, **caractérisé en ce que** les adducts contiennent à côté des groupes fonctionnels participant à la réaction de métathèse au moins un autre groupe qui contient des substituants se comportant de manière inerte dans la réaction de métathèse et/ou un hétéroatome.

5. Procédé selon la revendication 4, **caractérisé en ce que** lesdits substituants ou hétéroatomes sont choisis indépendamment parmi : des radicaux alkyle ramifiés ou non ramifiés, des cycles carbocycliques aromatiques ou non aromatiques, des acides carboxyliques, des esters, des éthers, des époxydes, des silyléthers, des thioéthers, des thioacétals, des anhydride, des imines, des éthers de silylènol, des sels d'ammonium, des amides, des nitriles, des groupes perfluoroalkyle, des groupes gem.-dialkyle, des alcynes, des alcènes, des halogènes, des alcools, des cétones, des aldéhydes, des carbamates, des carbonates, des uréthanes, des sulfonates, des sulfones, des sulfonamides, des groupes nitro, des unités organosilane, des centres métalliques, des hétérocycles contenant de l'oxygène, de l'azote, du soufre, du phosphore.

6. Procédé selon la revendication 1, **caractérisé en ce qu'**on utilise comme adducts des α,ω-diènes, qui peuvent contenir au moins un autre substituant se comportant de manière inerte dans la réaction de métathèse et/ou un hétéroatome, ces substituants ou hétéroatomes pouvant être choisis indépendamment parmi des radicaux alkyle ramifié ou non ramifiés, des cycles carbocycliques aromatiques ou non aromatiques, des acides carboxyliques, des esters, des éthers, des époxydes, des silyléthers, des thioéthers, des thioacétals, des anhydrides, des imines, des éthers de silylènol, des sels d'ammonium, des amides, des nitriles, des groupes perfluoroalkyle, des groupes gem.-dialkyle, des alcynes, des alcènes, des halogènes, des alcools, des cétones, des aldéhydes, des carbamates, des carbonates, des uréthanes, des sulfonates, des sulfones, des sulfonamides, des groupes nitro, des unités organosilanes, des centres métalliques, des hétérocycles contenant de l'oxygène, de l'azote, du soufre, du phosphore.

7. Procédé selon la revendication 6, **caractérisé en ce qu'**on utilise des α,ω-diènes, qui portent en position α d'une double liaison un substituant NRR¹, où
R est un substituant organique, de préférence l'hydrogène, un radical aryle éventuellement annelé, alkyle, CN, COOR² ou un halogène,
R¹ est un radical *tert*.-butyle, P(R)₂, P(R²)₂, COR, SO₂PhR, COOR ou CONRR²,
R² est un radical alkyle ou phényle,
R et R¹ signifient ensemble 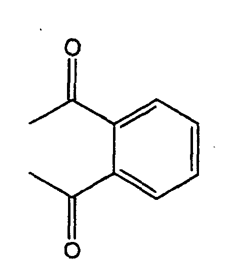 et lesdits α,ω-diènes peuvent également porter sur chaque autre position dans la molécule, à l'exception de la position α, au moins un autre substituant R.

8. Procédé selon la revendication 7, **caractérisé en ce qu'**on utilise les α,ω-diènes de formule générale (I) 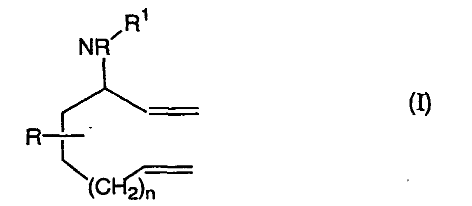 dans laquelle R, R¹ et R² qui ont la signification donnée dans la revendication 7 et n signifie les nombres 1,2,3 ou 4, de préférence 1 ou 2, en particulier de préférence 1.

9. Procédé selon la revendication 7, **caractérisé en ce qu'**on utilise comme α,ω-diènes une diallylamine ou un 3-amino-1,7-octadiène, en particulier de préférence dans leur forme N-carboxylméthyle protégée ou le 1,7-octadiène, le 10-undécénoyl-allylamide, le 1,4-bis-oxypropèn-yl-butène-2 ou l'ester 10-undécènoïquue-butèn-4-yle.

10. Procédé selon une ou plusieurs des revendications précédentes 1 à 9, **caractérisé en ce qu'**on peut utiliser comme catalyseurs des composés de formules générales (III) à (VI), dans lesquelles M signifie le ruthénium ou l'osmium 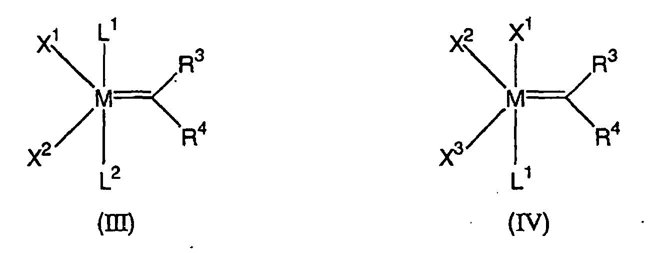 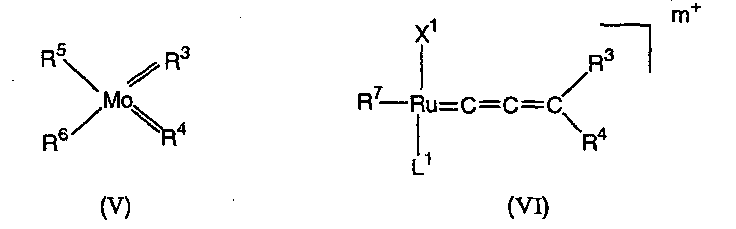 et
R³ à R⁷ indépendamment l'un de l'autre sont des radicaux pouvant être choisis parmi : l'hydrogène, un alkyle en C₁-C₂₀, cycloalkyle en C₃-C₈, alcényle en C₂-C₂₀, alcinyle en C₂-C₂₀, aryle en C₆-C₁₈, carboxylate en C₁-C₂₀, alcoxy en C₁-C₂₀, alcényloxy en C₂-C₂₀, alcinyloxy en C₂-C₂₀, aryloxy en C₆-C₁₈, alcoxycarbonyle en C₂-C₂₀, alkylthio en C₁-C₂₀, alkylsulfonyle en C₁-C₂₀ ou alkylsulfinyle en C₁-C₂₀, N-aryle ; chaque fois substitué au choix par un alkyle en C₁-C₁₂, perfluoroalkyle, halogène, alcoxy en C₁-C₅ ou aryle en C₆-C₁₈, où R³ à R⁷ peuvent également exister liés les uns avec les autres dans de composés cycliques,
X¹ à X³ sont des ligands anioniques pouvant être choisis indépendamment les uns des autres, en particulier F, Cl⁻, Br⁻, CN⁻, SCN⁻, R³O⁻, R³R⁴N⁻, (R³ - R⁷)-allyl⁻, (R³ - R⁷)-cyclopentadiényl⁻, où les radicaux R³ à R⁷ ont la définition déjà citée,
L¹ à L³ sont des ligands neutres pouvant être choisis indépendamment les uns des autres, en particulier CO, CO₂, R³NOO, R³R⁴C=CR⁵R⁶, R³C≡CR⁴, R³R⁴C=NR⁵, R³C≡N, R³OR⁴, R³SR⁴, NR³R⁴R⁵, PR³R⁴R⁵, AsR³R⁴R⁵, SbR³R⁴R⁵, où les radicaux R³ à R⁵ ont la définition déjà citée,
et
m est égal à 1 ou 2.

11. Procédé selon la revendication 10, **caractérisé en ce qu'**on utilise comme catalyseurs ou comme précurseurs de catalyseurs des composés de formules générales (III) et/ou (IV) avec L¹ et L² = PR³R⁴R⁵, où R³ à R⁵ correspondent aux définitions données ci-dessus, des radicaux tout particulièrement préférés sont en outre des radicaux aryle ou alkyle, en particulier des radicaux alkyle ou des radicaux cycloalkyle.

12. Procédé selon la revendication 11, **caractérisé en ce qu'**on utilise comme catalyseurs les composés suivants :

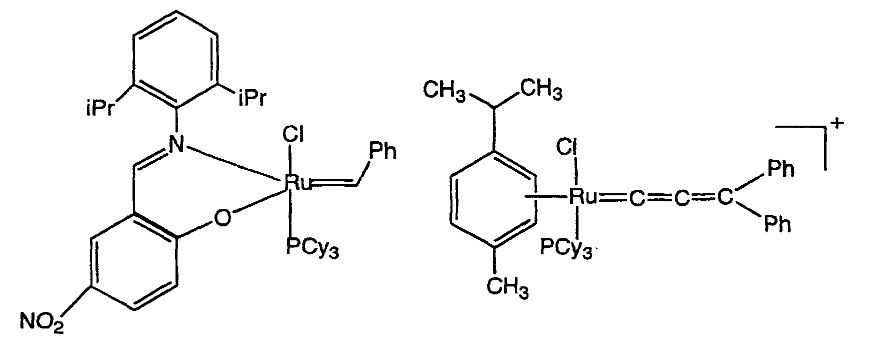

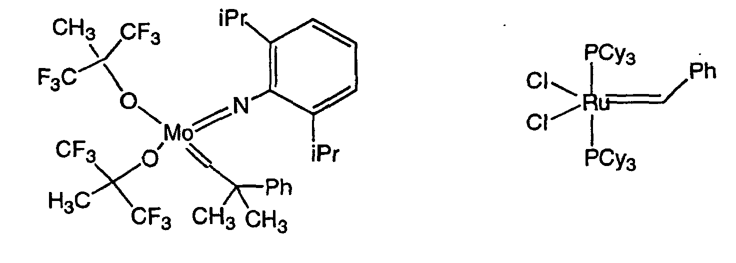

13. Procédé selon une ou plusieurs des revendications précédentes 1 à 12, **caractérisé en ce qu'**on utilise comme liquides ioniques l'hexafluorophosphate d'ammonium, le tétrafluoroborate d'ammonium, le tosylate d'ammonium ou l'hydrogénosulfate d'ammonium ou des mélanges de sels qui contiennent des halogénures d'aluminium en combinaison avec au moins un halogénure d'ammonium quaternaire et/ou au moins un halogénure de phosphonium quaternaire.

14. Procédé selon la revendication 13, **caractérisé en ce qu'**on utilise comme liquides ioniques l'hexafluorophosphate de pyridinium, le tétrafluoroborate de pyridinium, l'hydrogénosulfate de pyridinium, l'hexafluorophosphate de 1-méthyl-3-butylimidazolium ou des combinaisons de chlorure d'aluminium avec le chlorure de 1-méthyl-3-butylimidazolium, le chlorure de 1-méthyl-3-éthylimidazolium, le chlorure de N-butylpyridinium et/ou un halogénure de tétrabutylphosphonium.

15. Procédé selon une ou plusieurs des revendications précédentes 1 à 14, **caractérisé en ce qu'**on peut utiliser comme liquides ioniques également des combinaisons d'halogénure d'aluminium avec des mélanges d'halogénures d'ammonium quaternaire et/ou d'halogénures de phosphonium quaternaire, ainsi que des mélanges d'hexafluorophosphate d'ammonium, de tétrafluoroborate d'ammonium, de tosylate d'ammonium ou d'hydrogénosulfate d'ammonium.

16. Procédé selon une ou plusieurs des revendications précédentes 1 à 15, **caractérisé en ce que** le milieu réactionnel contient en outre un ou plusieurs additifs, qui sont choisis indépendamment parmi : des composés de phosphore, des amines, des composés perfluorés, des oxydes métalliques ou des solvants organiques.
